# EUROPEAN PATENT APPLICATION

(11) **EP 4 382 910 A1**
(43) Date of publication of application: **12.06.2024**
(21) Application number: 23198406.3
(22) Date of filing: 19.09.2023
(51) Int. Cl.: G01N 33/564, G01N 33/68, G01N 33/49

(54) **RAPID SCREENING METHODS FOR THYROID ASSOCIATED ORBITOPATHY**

(30) Priority: 07.12.2022 US 202263430793 P
(71) Applicant: The Chinese University Of Hong Kong, Shatin, New Territories (HK)
(72) Inventor: CHU, Wai Kit, TSEUNG KWAN O (HK); CHONG, Kam Lung Kelvin, SHATIN (HK)
(74) Representative: Gregson, Anna Louise

(57) **Abstract**

The present invention provides rapid screening methods, as well as related compositions and kits, for the detection of thyroid associated orbitopathy (TAO) by way of assessing the expression status of insulin-like growth factor 1 receptor (IGF-1R) on the surface of peripheral blood mononuclear cells (PBMCs) taken from an individual being examined for possible TAO.

## Description

### RELATED APPLICATIONS

This application claims priority to U.S. Provisional Patent Application No. 63/430,793, filed December 7, 2022, the contents of which are hereby incorporated by reference in the entirety for all purposes.

### BACKGROUND OF THE INVENTION

Graves' Disease (GD) is the commonest autoimmune syndrome. In the United States, about 1 in 100 Americans are affected by this disease. An autoimmune disorder, GD can cause hyperthyroidism, or an overactive thyroid gland. 25-50% of GD patients may suffer from Thyroid Associated Orbitopathy (TAO), which causes orbital deformities, ocular discomfort, and visual dysfunction. Patients with GD and TAO receive autoantibody assays to confirm, stage, and monitor the disease. Currently available antibody assays are laborious, however, and the test performance are affected by the quality of cultured cells specifically designed for the assays. In particular, these antibodies are thyroid-based but not specific for orbital diseases. No simple, reproducible, and reliable TAO screening test is currently available. Given the high prevalence of the GD and therefore TAO, there exists a pressing need to develop new and effective diagnostic methods that can provide rapid screening for TAO. The present invention fulfills this and other related needs.

### BRIEF SUMMARY OF THE INVENTION

The application provides the first disclosure of a rapid screening assay involving a simple blood cell staining method to differentiate TAO patients from healthy subjects. New compositions and related methods for diagnosing and treating TAO are therefore devised from this discovery.

As such, in a first aspect, this invention provides a method for assessing the status of insulin-like growth factor 1 receptor (IGF-1R) expression in peripheral blood mononuclear cells (PBMCs) in an individual. The method includes a first step of detecting, in a blood sample taken from the individual, PBMCs that express IGF-1R on the cell surface, and a second step of determining the percentage of PBMCs that express IGF-1R among all PBMCs in the blood sample. In some embodiments, the blood sample is whole blood sample. In some embodiments, the blood sample is all blood cell sample or a sample of the cellular portion of the blood. In some embodiments, the method also includes a step of comparing the percentage as determined from step (ii) to a standard control value. In some embodiments, the standard control value reflects the average percentage of IGF-1R expressing PBMCs among all PBMCs in healthy individuals, especially those who do not have GD or TAO and/or are at risk of developing GD or TAO. In some embodiments, the method further includes a step of determining the percentage from step (ii) being higher than the standard control value and determining the individual as suffering from TAO. In some cases, the individual may be subject to further clinical tests to confirm the diagnosis of TAO, for example, tests for presence of autoantibodies such as thyroid stimulating immunoglobulins, thyroid blocking immunoglobulins, and thyrotrophin binding-inhibiting immunoglobulins. In some cases, the individual, upon receiving the TAO diagnosis, receives therapeutic intervention such as application of eye drops/lubricant, administration of immunosuppressant including systemic steroids, orbital radiotherapy or surgical intervention.

In some embodiments, the method further includes, prior to step (i), a step of isolating PBMCs from a blood sample taken from the individual. Optionally, the method may further include an earlier step in which a blood sample is obtained from the individual. In some embodiments, step (i) comprises contacting the PBMCs with an antibody that specifically binds IGF-1R under suitable conditions so as to permit the antibody to specifically bind IGF-1R expressed on the cell surface of PBMCs. Optionally, the anti-IGF-1R antibody is attached to a detectable moiety to facilitate detection. In some embodiments, a secondary antibody that specifically binds the anti-IGF-1R antibody (and is optionally labeled with a detectable moiety) is used to facilitate detection of the anti-IGF-1R antibody. In some embodiments, step (i) is performed by using flow cytometry. In some embodiments, step (ii) is performed by using flow cytometry. In some embodiments, the individual being tested is a human. In some embodiments, the individual has been diagnosed with GD. In some embodiments, the individual has not been diagnosed with GD but may be at risk of later developing the disease.

In a second aspect, the present invention provides a kit for assessing IGF-1R expression status in PBMCs. The kit includes (1) a container containing a composition comprising an agent for detecting PBMCs that express IGF-1R on the cell surface; and (2) a standard control indicating the average percentage of IGF-1R-expressing PBMCs among all PBMCs in healthy subjects who do not have GD or TAO and/or who are deemed not at risk of later developing GD or TAO. In some embodiments, the agent comprises an anti-IGF-1R antibody that specifically binds IGF-1R. Optionally, the anti-IGF-1R antibody is attached to a detectable moiety to facilitate detection. In some embodiments, the kit may further include an agent for detecting the antibody. For example, a secondary antibody that specifically binds the anti-IGF-1R antibody (and is optionally labeled with a detectable moiety) is included in the kit to facilitate detection of the anti-IGF-1R antibody. In some embodiments, the kit further includes an agent for use in flow cytometry. Optionally, the kit may include an instruction manual directing the user for use of the kit.

In a related aspect, the present invention provides a new use for one or more agents capable of detecting PBMCs expressing IGF-1R on their cell surface in the manufacture of a kit described herein and above. Such agent(s) may include an antibody against IGF-1R.

### BRIEF DESCRIPTION OF THE DRAWINGS

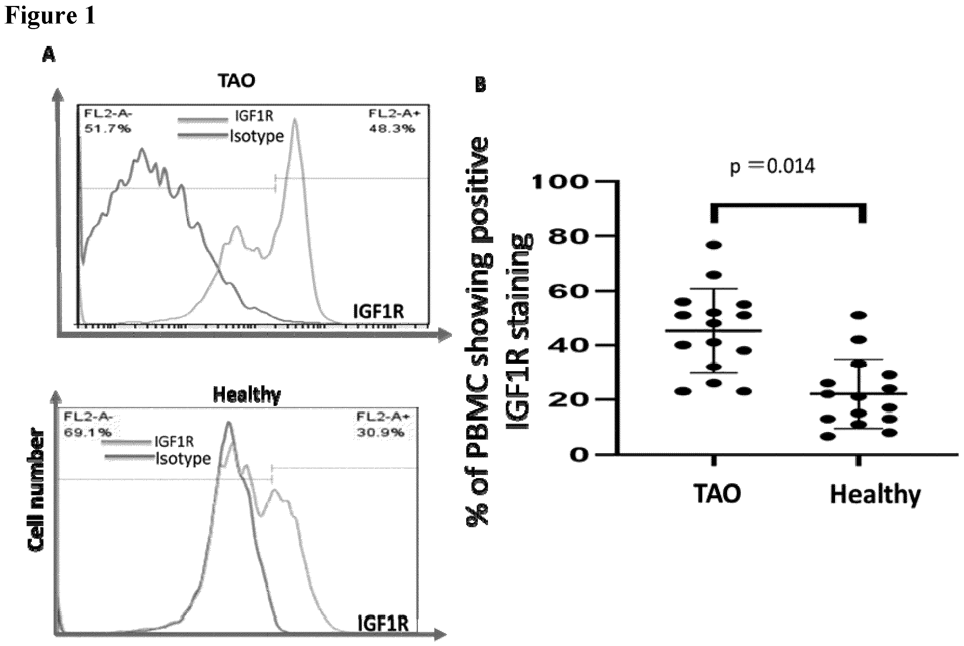

**Figure 1(A)** Representative of IGF1R staining and isotype antibody control staining in PBMC isolated from healthy and TAO patients. **Figure 1(B)** Percentage of PBMC showing positive staining of IGF1R in the healthy and TAO patients. Individual patient data points, means and standard deviations ae shown. Statistics was done by using T test.

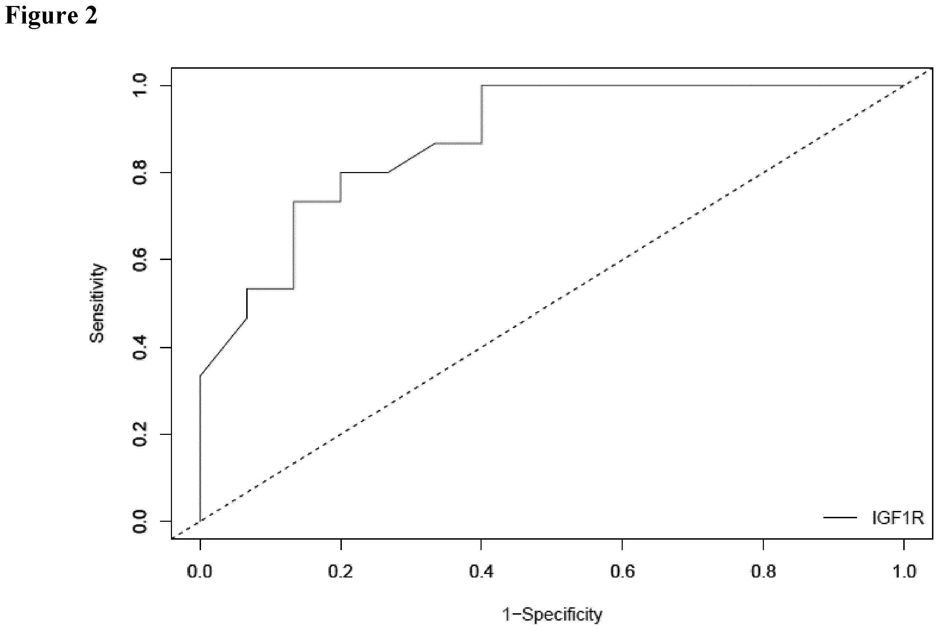

**Figure 2** Receiver Operating Characteristic (ROC) curve analysis of IGF1R staining in PBMC isolated from healthy and TAO patients (n=15 in both groups). Area Under Curve (AUC) is 87.778%. By using the Youden Index analysis, the cutoff value to separate healthy and TAO patients is determined at 32% of PBMC showing positive staining of IGF1R. By using this cutoff value, the sensitivity and specificity are both 0.8 and the positive predictive value is 71.4%.

### DEFINITIONS

The term "**insulin-like growth factor 1 receptor**" or "**IGF-1R**" refers to a protein expressed on the surface of cells, such as human cells. It is a transmembrane receptor that is activated by a hormone called insulin-like growth factor 1 (IGF-1) and by a related hormone IGF-2. IGF-1R belongs to the large class of tyrosine kinase receptors and mediates the effects of IGF-1, which is a polypeptide protein hormone similar in molecular structure to insulin, playing an important role during growth and mediating anabolic effects in humans. As used herein, the term IGF-1R encompasses human IGF-1R as well as homologues or orthologues of this protein in other species with at least 80%, 85%, 90%, 95% or higher sequence homology to any one of the exemplary human IGF-1R amino acid sequences and retaining the same or similar biological activity. The term also includes all variants of the gene product (especially human IGF-1R) due to alternative splicing. Human IGF-1R gene is located on chromosome 15: 98.65-98.96 Mb. Exemplary human IGF-1R amino acid sequences are provided as P08069 (UniProt), NP_000866 and NP_001278787 (GenBank).

The term "**Graves' Disease**" or "**GD**" refers to an immune system disorder that results in the overproduction of thyroid hormones or hyperthyroidism due to the presence of thyrotropin receptor (TSH-R) autoantibodies. Although GD may affect anyone, it is more common among women and in people younger than age 40 with increased risk when family history and/or other autoimmune diseases are present. The relevant symptoms related to hyperthyroidism include weight loss, despite an increased appetite; rapid or irregular heartbeat; nervousness, irritability, trouble sleeping, fatigue; shaky hands, muscle weakness; sweating or trouble tolerating heat. A substantial percentage of GD patients also develop an eye disease known as "Graves' ophthalmopathy (GO)," "thyroid-associated orbitopathy (TAO)," or "thyroid eye disease (TED)," which occurs when the immune system attacks the muscles and other soft tissues around the eyes. Symptoms can include bulging eyes, gritty or irritated eyes, puffy eyes, light sensitivity, pressure or pain in the eyes, and blurred or double vision. These symptoms can start before, at the same time, or after the onset of symptoms of hyperthyroidism. Rarely, the condition can develop after Graves' disease has been treated. While GD is usually treated with radioiodine therapy, medications (*e.g.*, antithyroid drugs carbimazole, methimazole, and propylthiouracil/PTU), and thyroid surgery, GO/TAO/TED is treated with lubricant eye drops or nonsteroidal anti-inflammatory drops (mild cases), steroids or orbital decompression (severe cases of corneal exposure or optic nerve compression), prism glasses and surgery (double vision), orbital decompression (for bulging eyes to retreat back into the head), surgeries on eyelids or bones around or behind the eyes (to alleviate the appearance of GO/TAO/TED). Cessation of smoking is recommended for patients.

The term "**peripheral blood mononuclear cell**" or "**PBMC**" refers to the blood cell type having a round nucleus. These PBMCs consist of lymphocytes (T cells, B cells, natural killer cells), monocytes, and dendritic cells, excluding erythrocytes and platelets, which have no nuclei, and granulocytes (neutrophils, basophils, and eosinophils), which have multi-lobed nuclei.

An "**antibody**" refers to a polypeptide substantially encoded by an immunoglobulin gene or immunoglobulin genes, or fragments thereof, which specifically bind and recognize an analyte (antigen). The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively.

An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

Antibodies exist, *e.g.,* as intact immunoglobulins or as a number of well characterized fragments produced by digestion with various peptidases. Thus, for example, pepsin digests an antibody below the disulfide linkages in the hinge region to produce F(ab)'₂, a dimer of Fab which itself is a light chain joined to V_{H}-C_{H}1 by a disulfide bond. The F(ab)'₂ may be reduced under mild conditions to break the disulfide linkage in the hinge region, thereby converting the F(ab)'₂ dimer into an Fab' monomer. The Fab' monomer is essentially an Fab with part of the hinge region (*see*, Paul (Ed.) Fundamental Immunology, 3rd Edition, Raven Press, NY (1993)). While various antibody fragments are defined in terms of the digestion of an intact antibody, one of skill will appreciate that such fragments may be synthesized *de novo* either chemically or by utilizing recombinant DNA methodology.

Further modification of antibodies by recombinant technologies is also well known in the art. For instance, chimeric antibodies combine the antigen binding regions (variable regions) of an antibody from one animal with the constant regions of an antibody from another animal. Generally, the antigen binding regions are derived from a non-human animal, while the constant regions are drawn from human antibodies. The presence of the human constant regions reduces the likelihood that the antibody will be rejected as foreign by a human recipient. On the other hand, "humanized" antibodies combine an even smaller portion of the non-human antibody with human components. Generally, a humanized antibody comprises the hypervariable regions, or complementarity determining regions (CDR), of a non-human antibody grafted onto the appropriate framework regions of a human antibody. Antigen binding sites may be wild type or modified by one or more amino acid substitutions, e.g., modified to resemble human immunoglobulin more closely. Both chimeric and humanized antibodies are made using recombinant techniques, which are well-known in the art (*see, e.g*., Jones et al. (1986) Nature 321 :522-525).

Thus, the term "antibody," as used herein, also includes antibody fragments either produced by the modification of whole antibodies or antibodies synthesized *de novo* using recombinant DNA methodologies (e.g., single chain Fv, a chimeric or humanized antibody) that retain the ability to specifically bind the same intended target antigen.

A "**label**," "**detectable label**," or "**detectable moiety**" is a composition detectable by radiological, spectroscopic, photochemical, biochemical, immunochemical, chemical, or other physical means. For example, useful labels include radioisotopes such as ³²P, fluorescent dyes, electron-dense reagents, enzymes (*e.g*., as commonly used in an ELISA), biotin, digoxigenin, or haptens and proteins that can be made detectable, *e.g.*, by incorporating a radioactive component into a polypeptide or used to detect antibodies specifically reactive with the polypeptide. Typically a detectable label is a heterologous moiety attached to a probe or a molecule (*e.g*., a protein or nucleic acid) with defined binding characteristics (*e.g.*, a polypeptide with a known binding specificity or a polynucleotide), so as to allow the presence of the probe/molecule (and therefore its binding target) to be readily detectable. The heterologous nature of the label ensures that it has an origin different from that of the probe or molecule that it labels, such that the probe/molecule attached with the detectable label does not constitute a naturally occurring composition (*e.g.*, a naturally occurring polynucleotide or polypeptide sequence).

The term "**immunoassay**" describes an assay that uses an antibody to specifically bind an antigen (*e.g.*, human IGF-1R). The immunoassay is characterized by the use of specific binding properties of a particular antibody to identify, isolate, target, and/or detect the presence or quantity of the antigen.

The phrase "**specifically binds**," when used to describe the binding relationship between an antibody and its target antigen, refers to a binding reaction that is determinative of the presence of the antigen (*e.g.*, a polypeptide such as IGF-1R) in a heterogeneous population of proteins and other biologics. Thus, under designated immunoassay conditions, the specified antibodies bind to a particular polypeptide (*e.g.*, human IGF-1R) generating a signal at least two times the background and do not substantially bind in a significant amount to other polypeptides or other antigens present in the sample. Specific binding to an antibody under such conditions may require an antibody that is selected for its specificity for a particular protein. For example, polyclonal antibodies raised to a human IGF-1R having the exemplary amino acid sequence indicated herein, can be selected to obtain only those polyclonal antibodies that are specifically immunoreactive with that specific protein and not with other proteins, e.g., other members of the growth factor receptor family. This selection may be achieved by subtracting out antibodies that cross-react with non-target molecules. A variety of immunoassay formats may be used to select antibodies specifically binding or immunoreactive with a particular protein. For example, solid-phase ELISA immunoassays are routinely used to select antibodies specifically immunoreactive with a protein (*see, e.g*., Harlow & Lane, Antibodies, A Laboratory Manual (1988) for a description of immunoassay formats and conditions that can be used to determine specific immunoreactivity). Typically, a specific binding reaction will yield at least twice of the background signal or "noise" and more typically more than 5, 10, 20, 50, or up to 100 times the background.

The term "**decreasing**" or "**decrease**," as used herein, refers to any detectable negative effect on a target biological process, such as gene expression, protein synthesis or phosphorylation, cellular signal transduction, cell proliferation, metabolism, tumorigenicity, and metastatic potential *etc.* Typically, a decrease is reflected in a negative change of at least 10%, 20%, 30%, 40%, 50%, 60%, 70%, 80%, or 90% in a target process or signal (*e.g*., IGF-1R protein expression), when compared to a standard control value. In a similar fashion, the term "increasing" or "increase" is used to describe any detectable positive effect on a target biological process or signal, for example, presence of IGF-1R on the cell surface of PBMCs, such as a positive change of at least 10%, 25%, 50%, 75%, 100%, or as high as 2, 3, 4, 5, 6, 7, 8, 9 or up to 10 or 20 fold, when compared to a standard control value. Other terms indicating quantitative changes or differences from a comparative basis, such as "**more**," "**higher**," "**less**," and "**lower**," are used in this application in the same fashion as described above.

"**Standard control**" as used herein refers to a sample that comprises PBMCs, among which a predetermined percentage are expressing a detectable level of IGF-1R on the cell surface, and is therefore suitable for the use in a method of the present invention, in order to be compared with the relative amount (*e.g.*, percentage) of IGF-1R-positive PBMCs among all PBMCs that are present in a test sample. Such a sample serving as a standard control provides an average percentage of IGF-1R-positive PBMCs in the blood of an average, healthy person who is neither diagnosed with GD/TAO nor at risk of developing GD/TAO (*e.g.*, as indicated by familiar history or the presence of other autoimmune disease). This average percentage as established by the "standard control" is known as a "standard control value," serving as a threshold or cut-off value for the purpose of diagnosing TAO according to the methods of this invention.

The term "**average**," as used in the context of describing a healthy person who is does not have any thyroid disorder, especially does not have and will unlikely develop GD or TAO, refers to certain characteristics, such as the relative amount (*e.g.*, the percentage) of LGF-1R-positive PBMCs found in the woman's blood, that are representative of a randomly selected group of healthy individuals who have normal thyroid functions and have no increased susceptibility to any thyroid-related diseases or conditions. This selected group should comprise a sufficient number of healthy individual such that the average percentage of the PBMCs expressing IGF-1R among these individual reflects, with reasonable accuracy, the corresponding percentage of IGF-1R expressing PBMCs in the general population of healthy individuals. In addition, the selected group of individuals generally have the same or adequately similar background such as age, gender, ethnicity, and overall health status.

The term "**about**" denotes a range of +/- 10% of a pre-determined value. For example, "about 10" sets a range of 90% to 110% of 10, *i.e.*, 9 to 11.

### DETAILED DESCRIPTION OF THE INVENTION

### I. Introduction

The present invention provides a new method for assessing the status of IGF-1R expression among PBMCs in the blood from an individual. The relative quantity or percentage of the PBMCs expressing a detectable level of IGF-1R among all PBMCs found in a blood sample is indicative of the individual's likelihood of suffering from TAO, especially among individuals who have already received a diagnosis of GD or individuals who are not yet diagnosed with GD but have a distinct, heightened risk for GD, *e.g.*, with a family history of the disease and/or with a personal history of other immunological disorder including autoimmune disease(s).

Upon determining the relative quantity or percentage of IGF-1R-positive PBMCs among all PBMCs in a test subject, the relative quantity or percentage is then compared to a standard control value that represents the average percentage of IGF-1R-positive PBMCs in healthy individuals' blood. If a test subject's percentage is higher than that of the standard control value or a cut-off threshold, the subject is deemed to have TAO or have an increased risk of developing TAO at a later time. In such case, the patient may be subject to further testing in order to confirm the diagnosis of TAO, for instance, tests for presence of autoantibodies such as thyroid stimulating immunoglobulins, thyroid growth immunoglobulins, and thyrotrophin binding-inhibiting immunoglobulins, especially if the patient has not previously been diagnosed with GD. A patient who has been deemed to have TAO according to the method of the present invention may then receive appropriate therapeutic intervention to treat GD, such as radioiodine therapy, antithyroid medications (*e.g.*, carbimazole, methimazole, and propylthiouracil/PTU), and thyroid surgery. In some cases, the patient receives treatment for TAO with the goal to alleviate or eliminate the symptoms of TAO, to delay or avoid the onset of the symptoms, or to lessen the frequency or severity of such symptoms, including but not limited to, application of lubricant eye drops or nonsteroidal anti-inflammatory drops, administration of steroids, use of prism glasses and surgery, orbital decompression, surgeries on eyelids or bones around or behind the eyes, as well as cessation of smoking.

### II. Acquisition of Blood Samples and Peripheral Blood Mononuclear Cells

The present invention relates to analyzing the status of IGF-1R expression on the cell surface of PBMCs in an individual as a means to detect the presence and/or to monitor the progress of TAO, especially in an individual who has received a diagnosis of GD or who is at increased risk for later developing GD. Thus, the first steps of practicing this invention are to obtain a blood sample from an individual being tested and isolate PBMCs from the sample.

### A. Acquisition of Blood Samples

A blood sample is obtained from an individual to be tested for TAO, who may be a patient already received a diagnosis of GD or who may be a person not yet diagnosed with GD but has notable risk for the condition (*e.g.*, with a family history or with other immune disorders especially autoimmune disorders) for screening using a method of the present invention. Collection of blood from an individual is performed in accordance with the standard protocol hospitals or clinics generally follow. An appropriate amount of peripheral blood, *e.g.*, typically between 1, 2, 5 to 10, 20, or up to 50 ml, is collected and maybe stored according to standard procedure prior to further preparation.

### B. Preparation of Blood Samples

In humans, lymphocytes account for the majority of the PBMC population, followed by monocytes, and only a small percentage of dendritic cells. The PBMCs are extracted from whole blood or buffy coat samples using a hydrophilic colloid and density gradient centrifugation, which separates the blood into a top layer of plasma, followed by a layer of PBMCs and a bottom fraction of polymorphonuclear cells (such as neutrophils and eosinophils) and erythrocytes. The analysis of IGF-1R expression profile in the PBMCs according to the present invention may be performed using, *e.g.*, the whole blood, cellular fraction of the whole blood, or buffy coat sample. The methods for preparing these samples from peripheral blood are well known among those of skill in the art. For example, a draw of peripheral blood can be placed in a tube containing EDTA or a specialized commercial product such as Vacutainer SST (Becton Dickinson, Franklin Lakes, NJ) to prevent blood clotting, and blood cells can then be obtained from whole blood through centrifugation, which is typically, though not exclusively, conducted at an appropriate speed, *e.g.*, 1,500-3,000 x g. The cellular fraction, enriched in the buffy coat portion of whole blood, can be obtained following centrifugation of a whole blood sample from the test subject and removal of the plasma.

### C. Isolation of PBMCs

A peripheral blood mononuclear cell (PBMC) is a blood cell having a single round nucleus such as lymphocyte, monocyte, or macrophage. There are numerous known methods for isolating PBMCs from a whole blood sample. Two primary techniques are through the use of a density gradient centrifugation process or by leukapheresis.

Since cells have a specific density, the use of a density gradient centrifugation process separates the main cell populations, such as lymphocytes, monocytes, granulocytes, and red blood cells, throughout a density gradient medium. After centrifugation, the PBMC fraction will appear as a thin white layer at the interface between the plasma and the density gradient medium making it easy to remove the PBMC fraction. For example, the PBMCs can be extracted from whole blood using ficoll, a hydrophilic polysaccharide that separates layers of blood, followed by gradient centrifugation, which will separate the blood into a top layer of plasma, followed by a lower layer of PBMCs and then a fraction of polymorphonuclear cells (such as neutrophils and eosinophils) and finally a bottom layer of erythrocytes.

A leukapheresis machine is an automated device that separates the inflow of whole blood from the target PBMC fraction using high-speed centrifugation while returning the outflow material, such as plasma, red blood cells, and granulocytes, back to the blood donor. On average, leukapheresis provides 14 times more PBMCs from a single donor than the density gradient centrifugation process of whole blood. The sheer number of PBMCs within a Leukopak, the product of leukapheresis, makes them ideal for large-scale research studies or pre-clinical trials. Depending on the quality of the leukapheresis machine and the downstream research applications, further processing of a Leukopak may be necessary to remove residual red blood cells (*e.g.*, by RBC lysis) and granulocytes (*e.g.*, by density gradient centrifugation).

### III. Detection and Counting of IGF-1R-Expressing PBMCs

Upon being extracted from a blood sample taken from a test subject, the PBMCs are analyzed to assess whether or not they express IGF-1R on their cell surface. A variety of well-known immunological assays may be used for this purpose. In some embodiments, a sandwich assay can be performed by capturing a target polypeptide (*e.g.*, IGF-1R expressed on PBMC surface) from a test sample with an antibody having specific binding affinity for the target polypeptide. Optionally, the antibody is conjugated to a detectable moiety, such that the presence of the target polypeptide (and cells expressing the target polypeptide) then can be readily detected based on the detectable signal. Such immunological assays can be carried out using microfluidic devices such as microarray chips. A protein of interest (*e.g.*, human IGF-1R protein) can also be detected by an immunoassay involving a secondary antibody that is readily detectable. For instance, the presence of IGF-1R on the cell surface of PBMCs may be detected first by specific binding of IGF-1R to a murine IgG anti-human IGF-1R antibody, which is then detected by virtue of its specific binding to a rabbit anti-murine IgG antibody, a secondary antibody that is conjugated to a detectable moiety (for example, a label emitting a fluorescent or chemiluminescent signal) and therefore permits rapid, easy, and accurate quantitative identification.

A particularly effective means for detecting and counting PBMCs that express a measurable amount of IGF-1R on the cell surface is by the technology known as flow cytometry. Fluorescence-activated cell sorting (FACS) is a specialized type of flow cytometry. It provides a method for sorting a heterogeneous mixture of biological cells into two or more containers or collections, one cell at a time, based upon the specific light scattering and fluorescent characteristics of each cell. These characteristics are predetermined so as to enable the system to focus on the detecting, counting, and separating cells based on the relevant features of the target cells. The FACS instrument provides fast, objective and quantitative recording of fluorescent signals from individual cells as well as physical separation of cells of particular interest. Generally, a cell suspension is entrained in the center of a narrow, rapidly flowing stream of liquid. The flow is arranged so that there is a large separation between cells relative to their diameter. A vibrating mechanism causes the stream of cells to break into individual droplets. The system is adjusted so that there is a low probability of more than one cell per droplet. Just before the stream breaks into droplets, the flow passes through a fluorescence measuring station where the fluorescent character of interest of each cell is measured. An electrical charging ring is placed just at the point where the stream breaks into droplets. A charge is placed on the ring based on the immediately prior fluorescence intensity measurement, and the opposite charge is trapped on the droplet as it breaks from the stream. The charged droplets then fall through an electrostatic deflection system that diverts droplets into containers based upon their charge.

An antibody specific for a particular cell surface protein (*e.g*., IGF-1R) is associated to a fluorescent molecule (*e.g.*, by conjugating the antibody to a molecule capable of emitting a fluorescent signal of a known wavelength upon excitation) and then added to PBMCs isolated from a blood sample taken from a test subject, to provide fluorescence when the specific cells (IGF-1R-positive PBMCs) pass through a laser beam they are monitored. Droplets containing single cells are given a positive or negative charge, based on whether the cell has limited the fluorescently-tagged antibody or not. Droplets containing a single cell are then detected by an electric field into collection tubes according to their charge. Cells bearing a protein of interest are first labeled with an antibody capable of specifically binding the particular cell surface molecule and coupled to a fluorescent dye, like when in a narrow stream the individual cells pass a laser beam in single file, the fluorescence of each cell is measured. A vibrating nozzle then forms small droplets, each droplet containing a single cell, and the droplets are given a negative or positive charge based on whether the cell they contain is fluorescing. A strong electric field defects the various charged droplets into separate containers so that each container has a homogeneous population of cells eventually with respect to the cell surface molecule tagged along fluorescent antibody, for example, a homogeneous population of PBMCs expressing IGF-1R on their surface in one container v. a homogeneous population of PBMCs not expressing IGF-1R on their surface in another container. This process allows PBMCs to be rapidly and reliably sorted and counted, the percentage of IGF-1R-positive PBMCs among all PBMCs is therefore readily determined.

### IV. Establishing a Standard Control

In order to establish a standard control for practicing the methods of this invention, a group of healthy individuals, who do not have thyroid disorders, especially do not have GD or do not have known risk for GD, are first selected. These individuals are preferably of similar ages, which are within the appropriate age range roughly matching that of the test subjects who are screened for TAO using the methods of the present invention. Similarly, a standard control value of percentage of IGF-1R-positive PBMCs is established using samples from a group of healthy individuals.

The healthy status of the selected individuals are confirmed by well-established, routinely employed methods with an emphasis on confirming normal thyroid functions of the individuals, including but not limited to, checking for symptoms of hyperthyroidism include weight loss, rapid/irregular heartbeat, nervousness, irritability, trouble sleeping, fatigue, shaky hands, muscle weakness; sweating or trouble tolerating heat, *etc*., as well as performing pertinent tests for the presence of autoantibodies such as thyroid stimulating immunoglobulins (TSI), thyroid blocking immunoglobulins (TBI), and thyrotrophin binding-inhibiting immunoglobulins (TBII).

Furthermore, the selected group of healthy individuals serving as standard controls must be of a reasonable size, such that the average percentage of IGF-1R-expressing PBMCs among all PBMCs in the peripheral blood obtained from the group can be reasonably regarded as representative of the normal or average percentage of PBMCs that express IGF-1R on their cell surface of healthy individuals without thyroid abnormalities. Preferably, the selected group comprises at least 10, 12, 15, 20, or 25 individuals.

### VI. Kits and Devices

The invention provides compositions and kits for practicing the methods described herein to assess the IGF-1R expression profile on the surface of PBMCs in a subject, which can be used for various purposes such as detecting or diagnosing the presence of TAO, determining the risk of developing TAO at a later time, and monitoring the progression of TAO in a patient, who may or may not have been given a diagnosis of GD.

Kits for carrying out assays for detecting the IGF-1R expression on the cell surface of PBMCs typically include at least one anti-IGF-1R antibody useful for specific binding with the IGF-1R protein present on the PBMC surface. Optionally, this anti-IGF-1R antibody is labeled with a detectable moiety. The antibody can be either a monoclonal antibody or a polyclonal antibody. In some cases, the kits may include at least two different antibodies, one for specific binding to the IGF-1R protein (*i.e*., the primary antibody) and the other for detection of the primary antibody by specifically binding to the primary antibody (*i.e*., the secondary antibody), which is often attached to a detectable moiety, such as a fluorescent label.

Typically, the kits also include an appropriate standard control. The standard controls indicate the average percentage of IGF-1R-positive PBMCs among all PBMCs in healthy subjects not suffering from or at increased risk of any thyroid disorders including GD. In some cases such standard control may be provided in the form of a physical sample, whereas in other cases the standard control may be provided as a set value. In addition, the kits of this invention may provide instruction manuals to guide users in analyzing test samples and assessing the expression profile of IGF-1R on the surface of PBMCs in a test subject, which may then be used to assess the presence, risk, or state of TAO in a test subject.

In a further aspect, the present invention can also be embodied in a device or a system comprising one or more such devices, which is capable of carrying out all or some of the method steps described herein. For instance, in some cases, the device or system performs the following steps upon receiving a blood sample, *e.g*., a sample containing PBMCs and taken from a subject being tested for detecting TAO, assessing the risk of developing TAO at a later time, or monitored for progression of TAO: (a) determining in sample the relative amount or percentage of PBMCs expressing the IGF-1R protein on their surface; (b) comparing the relative amount or percentage with a standard control value; and (c) providing an output indicating whether TAO is present in the subject or whether the subject is at risk of developing TAO in the future, or whether there is a change, *i.e*., worsening or improvement, in the subject's TAO condition. In other cases, the device or system of the invention performs the task of steps (b) and (c), after step (a) has been performed and the relative amount or average from (a) has been entered into the device. Preferably, the device or system is partially or fully automated.

### EXAMPLES

The following examples are provided by way of illustration only and not by way of limitation. Those of skill in the art will readily recognize a variety of non-critical parameters that could be changed or modified to yield essentially similar results.

To assess whether an individual suffers from thyroid-associated orbitopathy (TAO), 1 ml of peripheral venous blood can be taken from each person in need of such testing, who may or may not have been diagnosed with Grave's Disease (GD). Peripheral blood mononuclear cells (PBMC) will be isolated from the blood and stained for the insulin-like growth factor 1 receptor (IGF-1R). The percentage of IGF1R-positive PBMC will be quantified by flow cytometry.

Peripheral blood is collected from each patient being tested and stored in ethylenediaminetetraacetic acid (EDTA)-containing tubes. 1 ml of whole blood is treated with 10 ml 1X RBC lysis buffer (0.15 M ammonium chloride, 1.5 mM 4-(2-hydroxyethyl)-1-piperazineethanesulfonic acid, 2 mM sodium bicarbonate, 1 mM tetrasodium ethylenediaminetetraacetic acid, pH 7.2) at room temperature for 10 minutes and then centrifuged at 1500 rpm for 5 mins. Upon completion of centrifugation, the supernatant is discarded. Pelleted cells are then fixed in 4% paraformaldehyde for 10 minutes and again centrifuged at 1500 rpm for 5 mins, and the supernatant is again discarded when centrifugation is finished. Fixed cells are permeabilized in 1% Triton X100 buffer and then stained with a murine anti-IGF1Rα antibody (recombinant monoclonal human immunoglobulin G1, clone REA271 from Miltenyi Biotec; against antigen IGFIR (CD221); molecular mass of antigen is 151 kDa) at 1: 1000 dilution for 2 hours, washed by PBS for 2 times, followed by staining with Cy3 anti-mouse monoclonal antibody at 1: 1000 dilution. After washing by PBS for three times, stained cells are suspended in 200 µl staining buffer and analyzed with the flow cytometry machine. A reading of about 32% or greater IGF1R-positive PBMC supports a diagnosis of TAO in the patient. Figure 1A is the representative of IGF1R staining and isotype antibody control staining in PBMC isolated from healthy and TAO patients. Figure 1B illustrates the percentage of PBMC showing positive staining of IGF1R in the healthy and TAO patients. Individual patient data points, means and standard deviations ae shown. Statistics was done by using T test. Figure 2 shows the Receiver Operating Characteristic (ROC) curve analysis of IGF1R staining in PBMC isolated from healthy and TAO patients (15 subjects were studied in both groups). Area Under Curve (AUC) is 87.778%. By using the Youden Index analysis, the cutoff value to separate healthy and TAO patients is 32% of PBMC showing positive staining of IGF1R. By using this cutoff value, a sensitivity of 0.8, specificity of 0.8, and positive predictive value of 71.4% were achieved.

All patents, patent applications, and other publications, including GenBank Accession Numbers or similar sequence identification numbers, cited in this application are incorporated by reference in the entirety of their contents for all purposes.

## Claims

1. A method for assessing insulin-like growth factor 1 receptor (IGF-1R) expression status in peripheral blood mononuclear cells (PBMCs) in a patient, comprising the steps of:
(i) detecting in a blood sample taken from the patient PBMCs that express IGF-1R; and
(ii) determining percentage of PBMCs that express IGF-1R among all PBMCs.

2. The method of claim 1, further comprising comparing the percentage from step (ii) to a standard control value.

3. The method of claim 2, further comprising determining the percentage from step (ii) being higher than the standard control value and detecting thyroid-associated orbitopathy (TAO) in the patient.

4. The method of any one of claims 1-3, further comprising, prior to step (i), isolating PBMCs from a blood sample taken from the patient.

5. The method of any one of claims 1-4, further comprising, prior to step (i), obtaining a blood sample from the patient.

6. The method of any one of claims 1-5, wherein step (i) comprises contacting the PBMCs with an anti-IGF-1R antibody that specifically binds IGF-1R.

7. The method of claim 6, wherein the anti-IGF-1R antibody is conjugated to a detectable label.

8. The method of claim 6, wherein step (i) comprises contacting the PBMCs with the anti-IGF-1R antibody and a second antibody that specifically binds the anti-IGF-1R antibody.

9. The method of any one of claims 1-8, wherein step (i) or (ii) comprises flow cytometry.

10. The method of any one of claims 1-9, wherein the patient is a human patient.

11. The method of claim 10, wherein the patient has been diagnosed with Graves' Disease (GD).

12. A kit for assessing insulin-like growth factor 1 receptor (IGF-1R) expression status in peripheral blood mononuclear cells (PBMCs), comprising:
(a) an agent to detect PBMCs that express IGF-1R; and
(b) a standard control to indicate percentage of PBMCs that express IGF-1R among all PBMCs in healthy subjects.

13. The kit of claim 12, wherein the agent comprises an anti-IGF-1R antibody that specifically binds IGF-1R.

14. The kit of claim 13, wherein:
(a) the anti-IGF-1R antibody is conjugated with a detectable label;
(b) the kit further comprises a secondary antibody that specifically binds the anti-IGF-1R antibody; and/or
(c) the kit further comprises an agent for detecting the anti-IGF-1R antibody.

15. The kit of any of claims 12-14, further comprising:
(a) an agent for use in flow cytometry; and/or
(b) an instruction manual for using the kit.
